# EUROPEAN PATENT APPLICATION

(11) **EP 2 353 512 A1**
(43) Date of publication of application: **10.08.2011**
(21) Application number: 11250116.8
(22) Date of filing: 02.02.2011
(51) Int. Cl.: A61B 17/00

(54) **Surgical retrieval apparatus**

(30) Priority: 03.02.2010 US 301137 P; 16.12.2010 US 969674
(71) Applicant: Tyco Healthcare Group LP, New Haven, CT 06511 (US)
(72) Inventor: Gell, Jennifer Rachel, Cambridge CB3 0NS (GB); Collier, Nicholas John, Cambridge CB25 9JG (GB); Fleming, Alistair Ian, Lower Cambourne Cambridgeshire CB23 6ER (GB)
(74) Representative: Soames, Candida Jane

(57) **Abstract**

A surgical retrieval apparatus having an elongate member and a support member extending from the elongate member movable between a collapsed insertion position and an expanded position. A retrieval bag extends from the support member and has a first end and a closed second end, the first end movable to an open configuration when the support member moves to the expanded position. A net is positioned within the retrieval bag and detachably connected thereto.

## Description

### BACKGROUND

This application claims priority from provisional application serial no. 61/301,137, filed February 3, 2010, the entire contents of which are incorporated herein by reference.

### 1. Technical Field

The present disclosure relates to a surgical containment apparatus. More particularly, the present disclosure relates to a specimen retrieval apparatus for use in minimally invasive surgical procedures.

### 2. Background of Related Art

In minimally invasive surgical procedures operations are carried out within the body by using elongated instruments inserted through small entrance openings in the body. The initial opening in the body tissue to allow passage of instruments to the interior of the body may be a natural passageway of the body, or it can be created by a tissue piercing instrument such as a trocar, or created by a small incision into which a cannula is inserted.

Because the tubes, instrumentation, and any required punctures or incisions are relatively small, the surgery is less invasive as compared to conventional surgical procedures in which the surgeon is required to cut open large areas of body tissue. Therefore, minimally invasive surgery minimizes trauma to the patient and reduces patient recovery time and hospital costs.

Minimally invasive procedures may be used for partial or total removal of body tissue or organs from the interior of the body, e.g. nephrectomy, cholecystectomy, lobectomy and other procedures including thoracic, laparoscopic and endoscopic procedures. During such procedures, it is common that a cyst, tumor, or other affected tissue or organ needs to be removed via the access opening in the skin, or through a cannula. Various types of entrapment devices have been disclosed to facilitate this procedure. In many procedures where cancerous tumors are removed, removal of the specimen in an enclosed environment is highly desirable to prevent seeding of cancer cells.

In minimally invasive thoracic surgery, access to the thoracic cavity is limited as well as maneuverability within the cavity as the access port is placed between the confined space between a patient's ribs. Such procedures, commonly referred to as video assisted thorascopic surgery (VATS), aim to reduce patient recovery time by accessing the thoracic cavity through the natural intercostal space without spreading the ribs as in open procedures. This restricted access can sometimes cause problems when removing large specimens. Moreover, in such procedures, e.g. thorascopic wedge resection and lobectomy, it is often necessary to remove a portion of the lung and retrieve it relatively intact for pathology. It is also important that the specimen be sufficiently contained to prevent seeding of cancer cells during manipulation and removal.

In designing such specimen retrieval instrumentation, a balance must be struck between the need to provide a retrieval apparatus with a strong enough containment bag to prevent tearing or rupture while providing sufficient rigidity to enable manipulation and removal. Another balance which needs to be achieved is to provide sufficient maneuverability while reducing tissue trauma, e.g. damaging lung tissue, during manipulation and removal. Additionally, the instrumentation on one hand should be able to be inserted through a small access incision or port while on the other hand able to accommodate a wide range of patient sizes and be able to easily remove large specimens and minimize risk of seeding.

It would therefore be advantageous to provide a specimen retrieval device for minimally invasive surgical procedures with increased maneuverability and which minimizes trauma to surrounding tissue and which successfully achieves the balance of competing factors enumerated above.

### SUMMARY

The present disclosure is directed to a surgical retrieval apparatus. The present disclosure provides in one aspect a surgical retrieval apparatus comprising an elongate member and a support member extending from the elongate member and movable between a collapsed insertion position and an expanded position. A retrieval bag extends from the support member and has a first end and a closed second end, the first end movable to an open configuration when the support member moves to the expanded position. A net is positioned within the retrieval bag and detachably connected thereto, the net configured to receive and withdraw a tissue specimen.

In some embodiments, the support member is pivotably attached to the elongate member. The net can be attached to an inner surface of the retrieval bag. In some embodiments, the net is detachably connected along a perforated area of the retrieval bag. The net preferably has a dimension smaller than the dimension of the tissue sample to compress the tissue sample during removal.

In another aspect, the present disclosure provides a surgical retrieval apparatus comprising an elongate member, a support member adjacent a distal portion of the elongate member, a specimen retrieval bag supported by the support member, and a tissue specimen compression member positioned within the retrieval bag, wherein the compression member provides a compression force on the tissue specimen during removal through an incision in a patient.

In some embodiments, the compression member comprises a mesh structure. The compression member is preferably detachably connected to the retrieval bag. In some embodiments, detachment of the compression member enables it to move to a smaller transverse cross-sectional dimension.

In another aspect, the present disclosure provides a method of retrieving a tissue specimen from a patient comprising:
a) providing a surgical retrieval apparatus having:
   a support member;
   a retrieval bag extending from the support member and having a first end and a closed second end, the first end movable between closed and open configurations; and
   a net positioned within the retrieval bag;
b) introducing the support member into a body cavity to enable movement from a first position to a second expanded position to move the first end of the retrieval bag into the open configuration;
c) moving the tissue specimen into the net through the first end of the retrieval bag; and
d) applying a force to the net to remove the specimen.

In some embodiments, the step of applying a force to the net detaches the net from the retrieval bag. The support member and retrieval bag can be insertable through a delivery tube for insertion through the incision.

In some embodiments, the step of introducing the support member includes the step of introducing the support member into the thoracic cavity.

### BRIEF DESCRIPTION OF THE DRAWINGS

Embodiments of the presently disclosed specimen retrieval apparatus are described hereinbelow with reference to the drawings wherein:
FIG. 1 is a perspective view of the specimen retrieval apparatus of the present disclosure in the collapsed insertion position;
FIG. 2 is perspective view of the specimen retrieval apparatus of the present disclosure showing the retrieval bag of Figure 1 in the expanded position;
FIG. 3 is a close up view of a portion of the retrieval bag of Figure 2;
FIG. 4 is a perspective view of the retrieval apparatus within a delivery tube positioned within an incision to access the body cavity;
FIG. 5 is a perspective view of the specimen retrieval bag being deployed within the body cavity and positioned adjacent a tissue specimen;
FIG. 6 is a perspective view of the retrieval bag in the open position and a grasper placing the tissue specimen in the retrieval bag;
FIG. 7 is a perspective view of the tissue specimen positioned in the retrieval bag and the bag being retracted toward the incision;
FIG. 8 is a perspective view of the retrieval bag retracted to the edge of the incision;
FIG. 9 is a perspective view of the retrieval net being withdrawn from the retrieval bag; and
FIG. 10 is a perspective view of the retrieval bag and net being withdrawn from the incision.

### DETAILED DESCRIPTION OF THE EMBODIMENTS

Embodiments of the present disclosure will now be described in detail with reference to the drawings, in which like reference numerals designate identical or corresponding elements in each of the several views. As used herein, the term distal refers to the portion of the instrument which is further from the user while the term proximal refers to that portion of the instrument which is closer to the user.

The surgical retrieval apparatus disclosed herein may find use in any procedure where access to the interior of the body is limited to a relatively small incision, with or without the use of a cannula, as in minimally invasive procedures. The devices herein may find particular use in minimally invasive thoracic surgery where access to the thoracic cavity is through a space located between adjacent ribs known as the intercostal space.

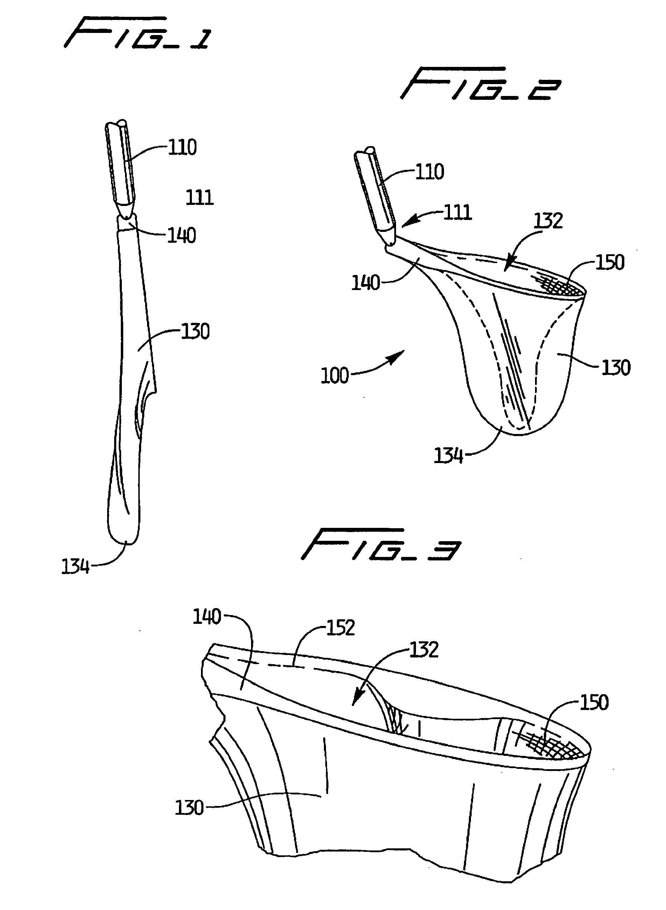

Referring initially to FIGS. 1 and 2, a surgical retrieval apparatus 100 is illustrated. Surgical retrieval apparatus 100 is preferably configured and dimensioned for use in minimally invasive surgical procedures (e.g. thoracic, laparoscopic, e ndoscopic, procedures). Surgical retrieval apparatus 100 includes an elongated tubular member 110 and a retrieval bag 130. The retrieval bag 130 is supported by a support member 140 in the form of a circumferential ring or rim. The rim 140 can be pivotably connected to the distal end 111 of tubular member 110 to enhance maneuverability of the retrieval bag within the cavity.

Positioned within the retrieval bag 130 is a net 150. The net 150 can be composed of a mesh of polymeric material, although other materials are also contemplated. For example, it can be formed of a molded plastic. The net 150 is preferably attached to the bag 130 along a perforation line 152 that extends along the upper periphery of the bag 130. Other locations of attachment as well as other methods of attachment are also contemplated. The net in a preferred embodiment can be substantially the same size as the bag 130. The net when attached to the bag 130 preferably has a diameter at its mouth or opening larger than the tissue specimen cross-section to facilitate entry of the specimen into the net and when detached portions of the net 150 can optionally have a diameter less than the transverse cross-section of the specimen, achieved for example by a taper, to prevent the tissue from falling to the bottom. In use, when a sufficient force is applied to the net 150 as it is pulled from the bag 130, the net detaches from the bag 130. This is described in more detail below in the method of use of the apparatus. Note the net 150 is elongated, having a length greater than its width.

The rim 140 can be composed of shape memory material with a shape memorized expanded position. Alternatively, it can be composed of other materials which enable collapse/compression of the ring for insertion and expansion for placement within the body cavity, such as materials exhibiting spring-like characteristics.

Once inserted inside the cavity, expansion of the rim 140 expands the mouth or opening 132 of bag 130 due to the attachment of the bag to the rim 140. The closed opposite end of the bag is designated by reference numeral 134. Expansion also expands the opening in the net 150 due to its attachment to the bag 130.

In use, the rim 140 and retrieval bag 130 can be delivered in a collapsed (e.g. folded) configuration through a delivery device 10 such as the configuration shown in Figure 1. As can be appreciated, by delivering the bag 130 in the collapsed configuration directly through a delivery tube 10 (without an external sleeve as part of the device positioned over the bag), the overall profile of the apparatus is minimized which enables a smaller diameter access port or delivery tube to be utilized. The device can also be delivered without a delivery tube directly through another access port or incision to reduce the profile and take up less space.

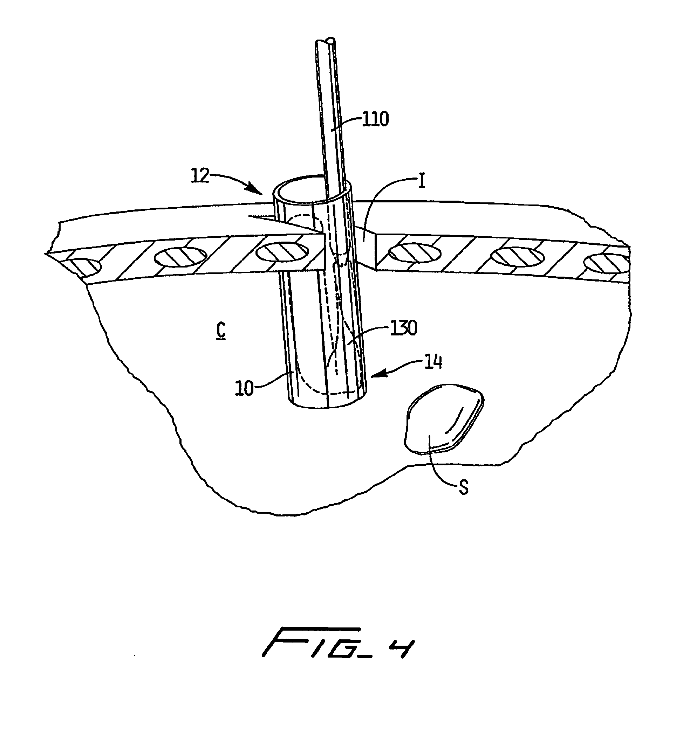

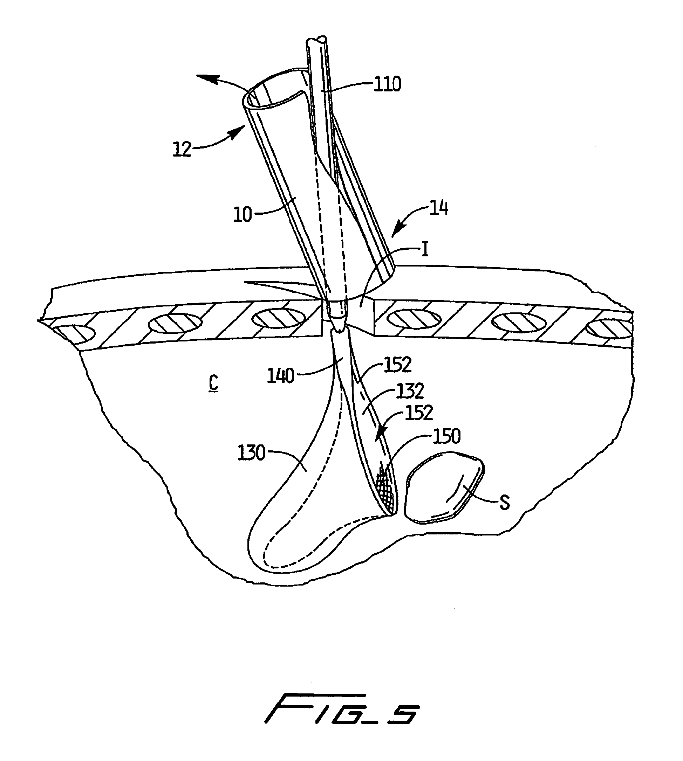

Turning now to Figures 4-10, the method of using the surgical apparatus to retrieve a specimen form the body cavity C, e.g. a thoracic cavity, will now be described. In the first step, delivery tube 10, containing the apparatus 100 in the collapsed or folded position, is placed through the incision I as shown in Figure 4, with the proximal portion 12 of delivery tube 10 extending outside the incision I and body cavity C and the distal portion 14 extending into the body cavity C. Next, the apparatus 100 is advanced from the delivery tube 10 by the surgeons advancing the tubular member 110 distally. Once the rim 140 is advanced past distal portion 115 of the delivery tube 10, (or the delivery tube 10 is retracted a sufficient distance to expose rim 140) the rim 140 automatically expands to a larger configuration as it is free from the confines of the wall of the delivery tube 10, thereby causing expansion of the bag 130 and opening of mouth 132 as shown in Figure 5 to present an opening for the tissue specimen S. Note that the bag opening 132 can be oriented toward the specimen by manipulation of the tubular member 110. Once the rim is exposed from the delivery tube 10, the delivery tube 10 is withdrawn over the proximal end of the tubular member 110. In one embodiment the tube 10 can have a peel away feature as shown in Figure 4 to allow separation of the tube 10 from the tubular member 110.

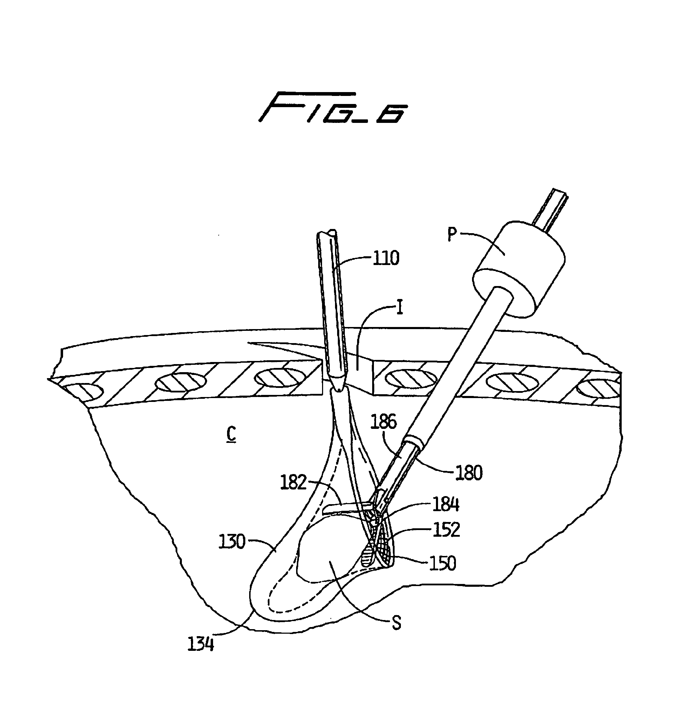

Next a grasper 180 is inserted through an access port P extending through a second incision as shown in Figure 6. The grasper 180 has a pair of jaws 182, 184 extending from shaft 186 and movable between open and closed positions to grasp the specimen S and place it through the opening 132 in the bag 130 and into the net 150 (through net opening 152) positioned therein. The grasper 180 can also be utilized to maneuver the bag 130 over the specimen S.

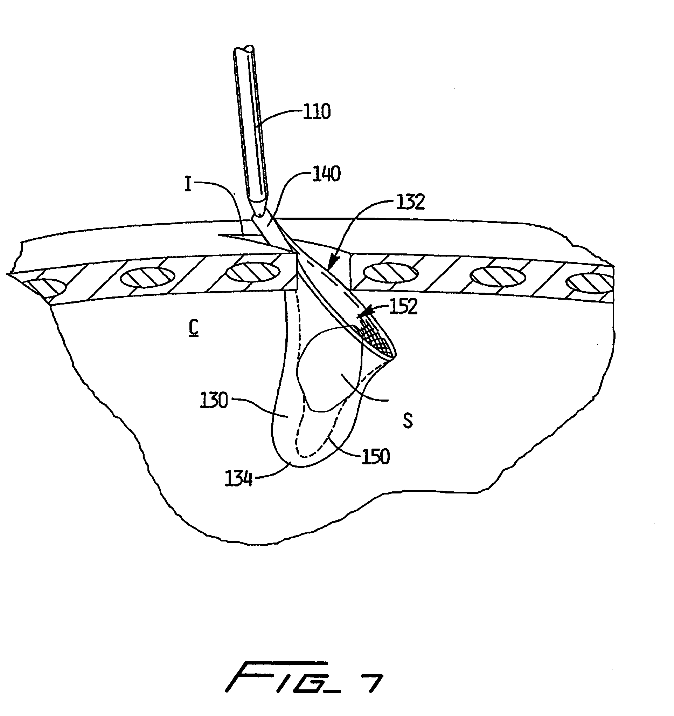

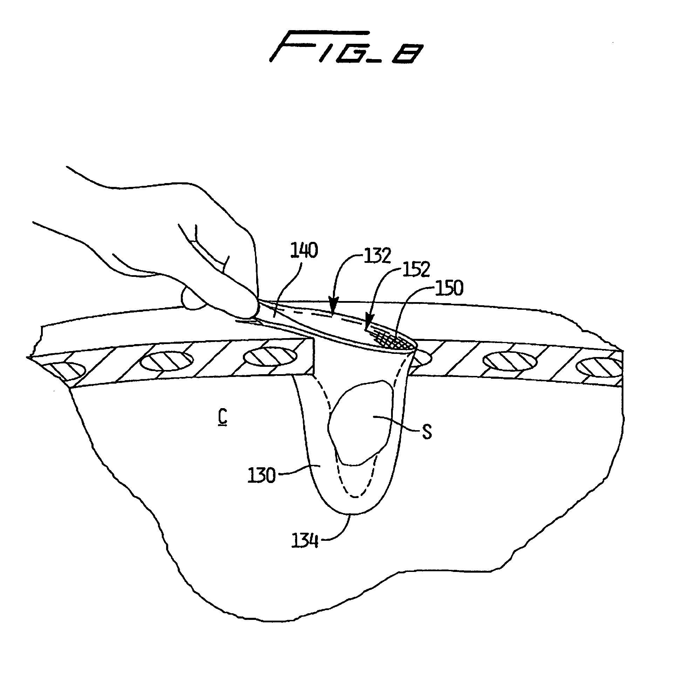

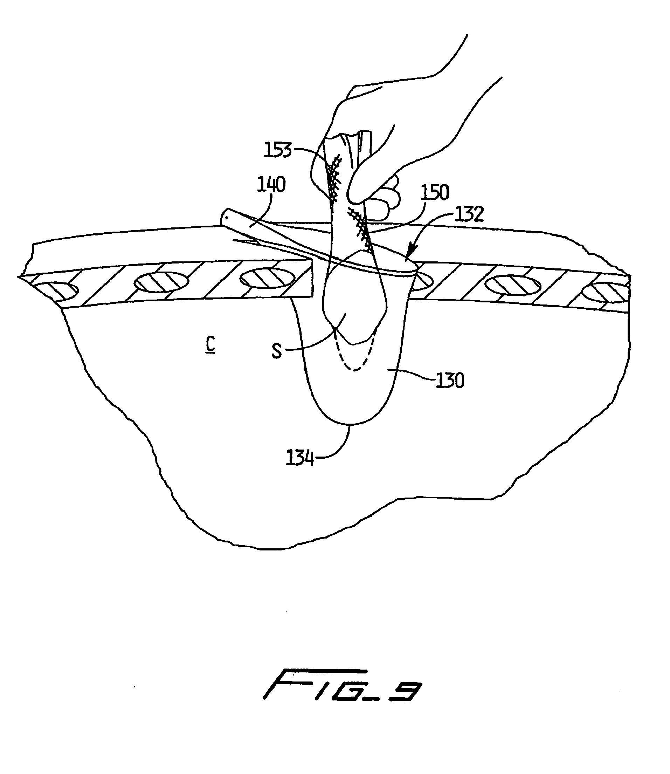

After placement of the specimen S in the net 150 within the bag 130, the grasper 180 is withdrawn leaving the specimen S within the net 150 as shown in Figure 7. The grasper 180 can be removed through the second incision at this time or removed after the specimen is removed. The tubular member 110 is then retracted to pull the opening 132 of bag 130 adjacent the incision I and partially up to the incision to the position of Figure 8 where the opening 132 in the bag 130 is at the incision for access by the user. This can be achieved by pulling further on the tubular member 110 or by grasping and pulling on the rim 140 as shown in Figure 8 after the tubular member 110 is detached from the rim 140. With such access, the user grasps a proximal portion 153 of the net 150 and applies a proximal retraction force (Figure 9), thereby severing the net 150 from its attachment to the bag 130 by detaching it along the perforation described above. With the net 150 now in its elongated and contracted position, detached from the bag 130 and therefore no longer expanded or stretched by the bag to a wider diameter (or transverse cross-section), the inner diameter of the net preferably being less than the diameter of the specimen S, it applies a compressive force to the specimen S. The net 150 thereby tightly holds the specimen S, compressing the specimen to maintain it in an elongated shape and prevent it from slipping to the bottom of the net 150 during removal.

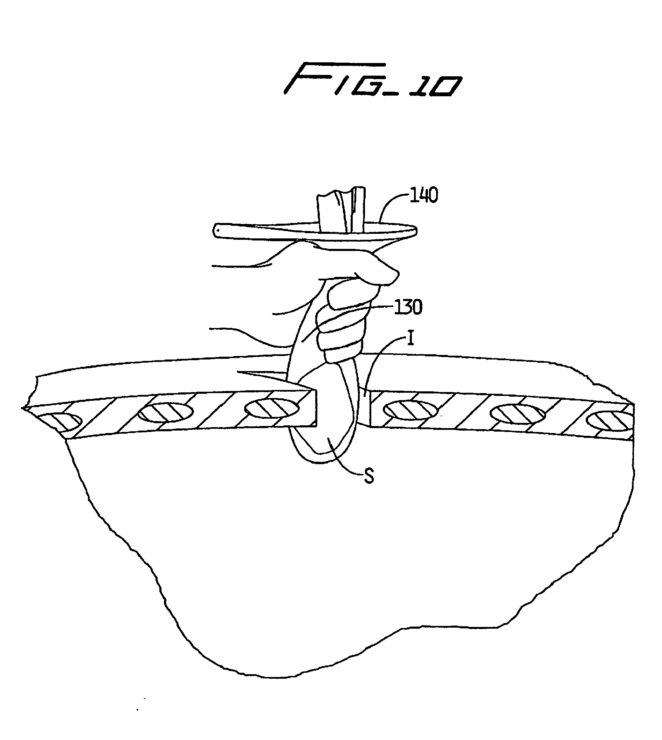

As shown in Figure 10, once the specimen is firmly held within the net 150, the user grasps the proximal portion of the bag 130 along with the net 150 and retracts it from the incision I. Note the net 150 can operate in a "Chinese finger" fashion so that applying a proximal retraction force on the net 150 elongates the net and applies additional compression force on the specimen S without damaging the specimen. The net can constrict as it is pulled out. The net can maintain a substantially oval-like shape of the specimen as it compresses it to better match the shape of the incision to facilitate removal.

Note the specimen can be maintained such that its long axis is substantially parallel to the incision which reduces the force required for removal through the incision or port. Also, as can be appreciated, the orientation and shape of the specimen S is substantially maintained to facilitate not only removal but pathology. Moreover, any compression or stretching of the specimen prior to removal occurs inside the bag which minimizes the risk of seeding.

Note also that the tissue specimen bags before containment so the risk of seeding is minimized. Also, since the net is taking the load, not the retrieval bag, the bag can be made of a thinner and lighter weight material and can be made more transparent.

As can be appreciated, the patient's body and cavity are shown schematically, it being understood that the surgical retrieval apparatus of the present disclosure can be used in the thoracic cavity, the abdominal cavity and other areas of the body for minimally invasive surgery.

A lubricious coating can be placed on the external surface of the specimen retrieval bag described herein to facilitate removal through the port or incision.

Markings can be provided along the length of the bag to indicate location of the tissue sample.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.

The invention may be described by reference to the following numbered paragraphs:
1. A surgical retrieval apparatus comprising an elongate member, a support member extending from the elongate member, the support member movable between a collapsed insertion position and an expanded position, a retrieval bag extending from the support member, the retrieval bag having a first end and a closed second end, the first end movable to an open configuration when the support member moves to the expanded position; and a net positioned within the retrieval bag and detachably connected thereto, the net configured to receive and withdraw a tissue specimen.
2. The surgical retrieval apparatus of paragraph 1, wherein the support member is pivotably attached to the elongate member.
3. The surgical retrieval apparatus of paragraph 1, wherein the net is detachably connected along a perforated area of the retrieval bag.
4. The surgical retrieval apparatus of paragraph 3, wherein the net is detachably connected to the retrieval bag along a perforation line that extends along an upper periphery of the bag.
5. The surgical retrieval apparatus of paragraph 1, wherein the net has a dimension smaller than the dimension of the tissue sample to compress the tissue sample during removal.
6. The surgical retrieval apparatus of paragraph 1, wherein the net is attached to an inner surface of the retrieval bag.
7. The surgical retrieval apparatus of paragraph 6, wherein the support member is pivotably attached to the elongate member.
8. The surgical retrieval apparatus of paragraph 1, wherein the detached net has a length greater than a width.
9. The surgical retrieval apparatus of paragraph 6, wherein the attachment of the net stretches the net to a wider transverse cross-section and detachment of the net enables the net to return to a narrower transverse cross-section.
10. A surgical retrieval apparatus comprising an elongate member, a support member adjacent a distal portion of the elongate member, a specimen retrieval bag supported by the support member, and a tissue specimen compression member positioned within the retrieval bag, the compression member providing a compression force on the tissue specimen during removal through an incision in a patient.
11. The surgical retrieval apparatus of paragraph 10, wherein the compression member comprises a mesh structure.
12. The surgical retrieval apparatus of paragraph 10, wherein the compression member is detachably connected to the retrieval bag.
13. The surgical retrieval apparatus of paragraph 12, wherein the compression member is connected along a perforated area of the retrieval bag.
14. The surgical retrieval apparatus of paragraph 10, wherein the compression member is attached to an inner surface of the retrieval bag.
15. The surgical retrieval apparatus of paragraph 12, wherein detachment of the compression member enables it to move to a smaller transverse cross-sectional dimension.
16. A method of retrieving a tissue specimen from a patient comprising a) providing a surgical retrieval apparatus having a support member, a retrieval bag extending from the support member and having a first end and a closed second end, the first end movable between closed and open configurations; and a net positioned within the retrieval bag; b) introducing the support member in to a body cavity to enable movement from a first position to a second expanded position to move the first end of the retrieval bag into the open configuration; c) moving the tissue specimen into the net through the first end of the retrieval bag; and d) applying a force to the net to remove the specimen.
17. The method of paragraph 16, wherein the step of applying a force to the net detaches the net from the retrieval bag.
18. The method of paragraph 17, wherein the support member and retrieval bag are insertable through a delivery tube for insertion through the incision.
19. The method of paragraph 16, wherein the step of introducing the support member into a body cavity includes the step of inserting the support member into the thoracic cavity.
20. The method of paragraph 16, wherein the support member is attached to an elongate member, and further comprising the step of manipulating the support member to position the first end of the retrieval bag adjacent the tissue specimen.

## Claims

1. A surgical retrieval apparatus comprising:
an elongate member;
a support member extending from the elongate member, the support member movable between a collapsed insertion position and an expanded position;
a retrieval bag extending from the support member, the retrieval bag having a first end and a closed second end, the first end movable to an open configuration when the support member moves to the expanded position; and
a net positioned within the retrieval bag and detachably connected thereto, the net configured to receive and withdraw a tissue specimen.

2. The surgical retrieval apparatus of claim 1, wherein the support member is pivotably attached to the elongate member.

3. The surgical retrieval apparatus of claim 1 or claim 2, wherein the net is detachably connected along a perforated area of the retrieval bag.

4. The surgical retrieval apparatus of claim 3, wherein the net is detachably connected to the retrieval bag along a perforation line that extends along an upper periphery of the bag.

5. The surgical retrieval apparatus of any preceding claim, wherein the net has a dimension smaller than the dimension of the tissue sample to compress the tissue sample during removal.

6. The surgical retrieval apparatus of any preceding claim, wherein the net is attached to an inner surface of the retrieval bag.

7. The surgical retrieval apparatus of claim 6, wherein the support member is pivotably attached to the elongate member.

8. The surgical retrieval apparatus of any preceding claim, wherein the detached net has a length greater than a width.

9. The surgical retrieval apparatus of claim 6, wherein the attachment of the net stretches the net to a wider transverse cross-section and detachment of the net enables the net to return to a narrower transverse cross-section.

10. A surgical retrieval apparatus comprising an elongate member, a support member adjacent a distal portion of the elongate member, a specimen retrieval bag supported by the support member, and a tissue specimen compression member positioned within the retrieval bag, the compression member providing a compression force on the tissue specimen during removal through an incision in a patient.

11. The surgical retrieval apparatus of claim 10, wherein the compression member comprises a mesh structure.

12. The surgical retrieval apparatus of claim 10 or claim 11, wherein the compression member is detachably connected to the retrieval bag.

13. The surgical retrieval apparatus of claim 12, wherein the compression member is connected along a perforated area of the retrieval bag.

14. The surgical retrieval apparatus of any of claims 10 to 13, wherein the compression member is attached to an inner surface of the retrieval bag.

15. The surgical retrieval apparatus of claim 12, wherein detachment of the compression member enables it to move to a smaller transverse cross-sectional dimension.
